(19) Europäisches Patentamt
European Patent Office
Office européen des brevets

(11) **EP 2 609 048 B1**

(12) **EUROPÄISCHE PATENTSCHRIFT**

(45) Veröffentlichungstag und Bekanntmachung des Hinweises auf die Patenterteilung:
**25.04.2018 Patentblatt 2018/17**

(21) Anmeldenummer: **11784928.1**

(22) Anmeldetag: **18.08.2011**

(51) Int Cl.:
*C03C 10/04* (2006.01)   *C03C 8/02* (2006.01)
*C04B 41/86* (2006.01)   *A61K 6/027* (2006.01)
*A61K 6/00* (2006.01)   *A61K 6/02* (2006.01)
*C04B 35/16* (2006.01)   *C03C 3/097* (2006.01)
*C03C 8/14* (2006.01)   *C04B 35/626* (2006.01)
*C04B 41/00* (2006.01)   *C04B 41/50* (2006.01)
*C04B 111/00* (2006.01)   *C04B 111/80* (2006.01)

(86) Internationale Anmeldenummer:
**PCT/DE2011/050026**

(87) Internationale Veröffentlichungsnummer:
**WO 2012/062292 (18.05.2012 Gazette 2012/20)**

(54) **VERBLENDKERAMIK FÜR DENTALE RESTAURATIONEN AUS YTTRIUMSTABILISIERTEM ZIRKONIUMDIOXID SOWIE VERFAHREN ZU DEREN AUFTRAG**

VENEERING CERAMIC FOR DENTAL RESTORATIONS MADE OF YTTRIUM-STABILIZED ZIRCONIUM DIOXIDE AND METHOD FOR APPLYING SAID VENEERING CERAMIC

CÉRAMIQUE DE REVÊTEMENT POUR RESTAURATIONS DENTAIRES À BASE DE DIOXYDE DE ZIRCONIUM STABILISÉ PAR L'YTTRIUM AINSI QUE SON PROCÉDÉ D'APPLICATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.08.2010 DE 102010035545**

(43) Veröffentlichungstag der Anmeldung:
**03.07.2013 Patentblatt 2013/27**

(73) Patentinhaber: **Fraunhofer-Gesellschaft zur Förderung der angewandten Forschung e.V.
80686 München (DE)**

(72) Erfinder:
• **EHRT, Roland**
  **07747 Jena (DE)**
• **JOHANNES, Martina**
  **07629 Hermsdorf (DE)**

(74) Vertreter: **Oehmke, Volker et al
Patentanwälte Oehmke & Kollegen
Neugasse 13
07743 Jena (DE)**

(56) Entgegenhaltungen:
**WO-A1-2011/076422      WO-A2-2008/106958
US-A1- 2005 127 544**

Bemerkungen:
Die Akte enthält technische Angaben, die nach dem Eingang der Anmeldung eingereicht wurden und die nicht in dieser Patentschrift enthalten sind.

## Beschreibung

Die Erfindung betrifft Verblendkeramiken für dentale Restaurationen, wobei die Gerüstkeramik aus yttriumstabilisiertem Zirkoniumdioxid besteht.

Die Eigenschaften der Verblendkeramiken werden durch die chemische Zusammensetzung, die Kristallphasen, die kristalline Struktur sowie der Körnung des Ausgangsmaterials bestimmt. Es besteht die Zielstellung, die hohen mechanischen, chemischen und ästhetischen Anforderungen an die Verblendkeramiken zu erfüllen und gleichzeitig den Fertigungsaufwand bei der Modellierung der Verblendkeramik zu reduzieren.

Für das Verblenden von dentalen Restaurationen wird der Stand der Technik durch leucithaltige Dentalkeramiken geprägt. Beim Patent US 4,798,536 A liegt der Leucitgehalt im Bereich von 35 bis 60 Gew %. Die Biegebruchfestigkeit der Verblendkeramik mit Leucitkristallen beträgt ca. 80 MPa.

Mit dem Patent US 4,515,634 A wird vorgeschlagen, im Grundsystem $Li_2O$-$CaO$-$Al_2O_3$-$SiO_2$ zur Verbesserung der Keimbildung und Kristallisation den Keimbildner $P_2O_5$ zuzusetzen.

In DE 197 25 552 A1 werden für keramische Dentalrestaurationen Alkali-Silicat-Gläser beschrieben. Die Dentalmaterialien enthalten bevorzugt Apatit - Kristalle. Bei der beschriebenen Apatit - Glaskeramik sollten die Komponenten $CaO$ : $P_2O_5$ : F in einem definiertem Mol - Verhältnis von 1 : 0,02 bis 1,5 : 0,03 bis 4,5 enthalten sein.

In der Patentschrift DE 10 2004 013 455 B3 werden Apatit - Glaskeramiken auf der Basis von phosphat- und fluorfreien silicatischen Oxyapatiten sowie Verfahren zu ihrer Herstellung und Verwendung beschrieben. Als Kristallphase kann neben den Oxyapatiten auch Leucit auftreten.

In der Patentschrift DE 196 47 739 C2 wird eine sinterbare Lithiumdisilicat-Glaskeramik sowie Glas beschrieben. Das Ausgangsmaterial wird zu Rohlingen gesintert. Diese Rohlinge werden bei 700°C bis 1200°C zu dentalen Produkten gepresst. Bei der plastischen Verformung weist die beschriebene Lithiumdisilicat - Glaskeramik nur eine geringe Reaktion mit der benachbarten Einbettmasse auf.

Die Offenlegungsschrift DE 197 50 794 A1 beschreibt den Einsatz von Lithiumdisilicat - Glaskeramiken für die Nutzung im Heißpressverfahren. Es hat sich jedoch gezeigt, dass bei der Anwendung dieses Verfahrens die Kantenfestigkeit am restaurierten Zahn nicht ausreichend ist und bei der Nachbearbeitung ein hoher Werkzeugverschleiß eintritt. In der US 2005/0127544 A1 wird ebenfalls eine gut pressbare Lithiumdisilicat-Glaskeramik beschrieben. Die über die Kristallisation eines kompakten Ausgangsglases hergestellte Glaskeramik weist eine Festigkeit von ca. 350 MPa auf. Dieser Glaskeramiktyp kann im Heißzustand zu dentalen Produkte wie Kronen, Brücken oder Veneers geformt werden. Darüber hinaus kann aus der Glaskeramik bzw. dem Ausgangsglas auch ein Pulver hergestellt werden. Das kristallisierte Pulver wird mit einer organischen Komponente (Composite) versehen und dient dem Verfüllen des Zahnes. In WO 2011/076422 A1 wird ebenfalls eine Gerüstkeramik auf Basis einer Lithiumdisilicat - Glaskeramik beschrieben. Dabei erfolgt die Kristallisation zweistufig. In der ersten Stufe wird das glasige Ausgangsmaterial in eine Lithiummetasilicat -Glaskeramik überführt und die gute mechanische Bearbeitung genutzt. Die Formgebung der Gerüstkeramik erfolgt über CAD/CAM. Über die zweite Temperaturbehandlung und die Reaktion mit dem in der glasigen Phase vorhandenen SiO2 kommt es zur Kristallumwandlung zu Lithiumdisilicat.

Mit der DE 103 36 913 A1 wird vorgeschlagen, den zu restaurierenden Zahn in zwei Stufen zu fertigen. In der ersten Stufe wird Lithiummetasilicat zur Kristallisation gebracht, das durch maschinelle Verarbeitung zu dentalen Produkten verarbeitet wird. In einer zweiten Temperaturbehandlung wird das Lithiummetasilicat in das festere Lithiumdisilicat überführt.

In der Patentschrift WO 2008/106958 A2 werden Verblendkeramiken mit hoher Festigkeit und einer guten Haftung zum yttriumstabilisierten Zirkoniumoxid beschrieben. Bei den beschriebenen Verblendkeramiken kommt es neben Lithiumdisilicat auch zur Kristallisation von ß-Spodumen, Lithiummetasilicat und unterschiedlicher Phasen vom Siliziumdioxid.

Auf der Grundlage von yttriumstabilisiertem Zirkoniumdioxid wird in der EP 1 235 532 A1 ein Verfahren zur Herstellung von hochfestem keramischen Zahnersatz beschrieben. Die nach diesem Verfahren gefertigten Gerüstkeramiken weisen 4-Punkt Biegebruchfestigkeiten größer als 1200 MPa auf.

Der Erfindung liegt die Aufgabe zugrunde, für dentale Restaurationen aus yttriumstabilisiertem Zirkoniumdioxid eine transluzente Verblendkeramik auf Basis von Lithiumdisilicat sowie ein Verfahren zur Aufbringung der Verblendkeramik zu entwickeln. Diese Verblendkeramik soll kein Leucit, Lithiummetasilicat oder β - Spodumen enthalten und eine sehr gute chemische Beständigkeit, eine hohe Haftfestigkeit zum Zirkoniumdioxid sowie eine gute Oberflächenqualität aufweisen.

Erfindungsgemäß wird diese Aufgabe bei einer Verblendkeramik auf yttriumstabilisiertem Zirkoniumdioxid dadurch gelöst, dass sie aus den folgenden Komponenten hergestellt wird:

    a) $SiO_2$ 55,0 - 72,5 Ma. %
    b) $Nb_2O_5$ 6,0 - 19,8 Ma. %
    c) $B_2O_3$ 1,0 - 9,0 Ma. %

d) $Al_2O_3$ 1,2 - 6,0 Ma. %
e) $Li_2O$ 5,0 - 16,5 Ma. %
f) $Na_2O$ 1,4 - 11,0 Ma. %
g) $ZrO_2$ 0,5 - 4,0 Ma. %

und auf der Oberfläche der Verblendkeramik mit einer während der Kristallisation ausgebildeten dünnen Glasschicht versehen wird.

Lithiumdisilicat bildet die Hauptkristallphase der Verblendkeramik. Es ist gewünscht, jedoch nicht zwingend notwendig, dass neben dem Lithiumdisilicat auch teilweise Nioboxid auskristallisiert wird. Nioboxid wird nicht bzw. nur in geringen Mengen in die Struktur vom Lithiumdisilicat eingebaut. Mittels gesteuerter Kristallisation werden niobreiche Phasen in unmittelbarer Nähe zum Lithiumdisilicat angereichert.

Die Änderung der Phasenzusammensetzung durch den Kristallisationsprozess führt zu nioboxidreichen Glasphasen. Die nioboxidreichen Glasphasen ermöglichen eine sehr hohe Oberflächenqualität der Verblendkeramik, so dass kein gesonderter Glanzbrand erforderlich ist.

Zur Farbanpassung bzw. Fluoreszenz können der Verblendkeramik färbende Elemente bzw. Pigmente zugesetzt werden. Die Konzentration der einzelnen Farbkomponenten sollte bei maximal 3,0 Ma. % liegen.

Zur Modifizierung der Technologie können unabhängig voneinander die Zusätze $La_2O_3$, BaO, CaO, MgO, ZnO, $P_2O_5$, $TiO_2$ und Fluorid zugegeben werden, wobei die Konzentration der angeführten Zusätze in deren Summe maximal im Bereich bis zu 4,0 Ma. % liegt.

Die Verblendkeramik weist eine sehr gute Haftung zum Zirkoniumdioxid auf. Deshalb ist der Einsatz eines Opakers bzw. Liners nicht zwingend notwendig.

Die angeführten Ausgangsmaterialien der Verblendkeramik können auf das yttriumstabilisierte Zirkoniumdioxid als Pulver mittels Schichttechnik aufgebracht werden. In einem definierten Mahlprozess können die Pulver auch zu einem Schlicker verarbeitet werden, welcher mittels Sprühen aufgebracht wird. Nach anschließender Kristallisation entsteht eine Verblendkeramik mit hoher Oberflächenqualität.

Die Verblendkeramik ermöglicht die Kombination der Temperaturbehandlung im Dentalofen mit einer nachträglichen Laserbehandlung. Dazu werden die auf die Gerüstkeramik aufgetragenen Ausgangsmaterialien der Verblendkeramik mittels Dentalofen in den Temperaturbereichen von 850°C bis 910°C bzw. 911°C bis 940°C aufgesintert. Die gesteuerte Kristallisation erfolgt im Temperaturbereich von 850°C bis 910°C, wobei die entstehende Verblendkeramik eine hohe Oberflächenqualität aufweist. Die so erzeugte Verblendkeramik kann bereits für dentale Restaurationen genutzt werden. Mittels Einsatz eines Lasers ist in einem weiteren Verfahrensschritt eine lokale Verglasung der Verblendkeramik möglich.

Ebenso ist es möglich, dass die Ausgangsmaterialien der Verblendkeramik im Temperaturbereich von 911°C bis 940°C auf die Gerüstkeramik aufgesintert werden und in einem weiteren Verfahrensschritt mittels Lasers eine lokale Kristallisation induziert wird.

Die Verblendkeramik kann in mehreren Schichten aufgetragen werden. Die sehr gute Haftung zum Zirkoniumdioxid und die sehr gute Oberflächenqualität ermöglichen es, dass bereits eine gesprühte Schicht für den Verblendungsprozess ausreichend ist.

Die Erfindung soll nachstehend anhand von Ausführungsbeispielen und Zeichnungen näher erläutert werden. Dabei zeigt:

Fig. 1 einen möglichen Temperaturverlauf zur Herstellung der Verblendkeramik,
Fig. 2 ein Röntgendiffraktogramm (XRD) der erfindungsgemäßen Verblendkeramik nach der Temperaturbehandlung bei 900°C,
Fig. 3 eine Gefügestruktur der Verblendkeramik auf Zirkoniumdioxid und
Fig. 4 einen Temperaturverlauf für die Herstellung der Verblendkeramik über laserinduzierte Kristallisation.

In der nachfolgenden Tabelle 1 sind typische Zusammensetzungen ausgewiesen.

**Tabelle 1**

| Beispiele | 1 | 2 | 3 | 4 | 5 | 6 | 7 | 8 | 9 |
|---|---|---|---|---|---|---|---|---|---|
| | Ma. % | Ma. % | Ma. % | Ma. % | Ma. % | Ma. % | Ma. % | Ma. % | Ma. % |
| $SiO_2$ | 62,5 | 65,6 | 56,2 | 65,4 | 65,5 | 64,5 | 61,5 | 63,8 | 64,2 |
| $Nb_2O_5$ | 19,0 | 15,4 | 18,2 | 10,8 | 13,1 | 12.1 | 6,7 | 9,9 | 12,8 |
| $B_2O_3$ | 1,1 | 1,4 | 3,0 | 2,7 | 1,4 | 1,8 | 6,5 | 3,0 | 1,5 |

(fortgesetzt)

| Beispiele | 1 | 2 | 3 | 4 | 5 | 6 | 7 | 8 | 9 |
|---|---|---|---|---|---|---|---|---|---|
| | Ma. % | Ma. % | Ma. % | Ma. % | Ma. % | Ma. % | Ma. % | Ma. % | Ma. % |
| $Al_2O_3$ | 1,4 | 1,5 | 4,3 | 2,5 | 2,0 | 3,0 | 4,7 | 4,0 | 1,9 |
| $Li_2O$ | 13,5 | 13,7 | 5,2 | 11,4 | 12,5 | 11,4 | 6,3 | 9,9 | 12,2 |
| $Na_2O$ | 1,5 | 1,4 | 9,5 | 5,3 | 4,0 | 4,8 | 10,5 | 6,1 | 3,9 |
| $ZrO_2$ | 1,0 | 1,0 | 3,6 | 1,9 | 1,5 | 1,6 | 3,8 | 2,0 | 1,5 |
| MgO | - | - | - | - | - | 0,8 | - | 1,3 | - |
| $TiO_2$ | - | - | - | - | - | - | - | - | 2,0 |
| | | | | | | | | | |
| Summe | 100 | 100 | 100 | 100 | 100 | 100 | 100 | 100 | 100 |

Für die dentale Restauration kommt als Gerüstkeramik yttriumstabilisiertes Zirkoniumdioxid zum Einsatz. Diese kann gezielt eingefärbt sein. Im Temperaturbereich von 100° bis 500°C hat das yttriumstabilisierte Zirkoniumdioxid einen thermischen Ausdehnungskoeffizienten $\alpha$ von 11,1 x 10$^{-6}$ / K.

Für die erfindungsgemäß beschriebenen Verblendkeramiken liegt der thermische Ausdehnungskoeffizient $\alpha_{100°C \text{ bis } 500°C}$ in den Grenzen zwischen

$$9,5 \times 10^{-6} / K \quad \text{und} \quad 11,0 \times 10^{-6} / K.$$

Die Bestimmung der Haftfestigkeit zwischen dem yttriumstabilisierten Zirkoniumdioxid und der Verblendkeramik erfolgte im Dreipunktbiegeversuch. Dazu wurde die gepulverte Verblendkeramik auf die Stirnfläche von zwei Rundstäben aus Zirkoniumdioxid aufgebracht und der definierten Temperaturbehandlung unterzogen.

Die nach dem beschriebenen Verfahren gemessene Haftfestigkeit sollte ≥ 100 MPa betragen.

Die chemische Beständigkeit wurde nach DIN EN 6872:2009-1 ISO bestimmt und ist bei der erfindungsgemäßen Verblendkeramik < 50 $\mu$g / cm$^2$.

In der Tabelle 2 sind die Kennwerte für Ausdehnungskoeffizient $\alpha_{100°C \text{ bis } 500°C}$, Haftfestigkeit und chemische Beständigkeit zu den Zusammensetzungen entsprechend der Tabelle 1 für die Beispiele 4 und 5 dokumentiert.

**Tabelle 2**

| Kennwerte | Beispiel 4 | Beispiel 5 |
|---|---|---|
| $\alpha_{100°C \text{ bis } 500°C}$ | 10,4 x 10$^{-6}$ / K | 10,1 x 10$^{-6}$ / K |
| Haftfestigkeit | 148 MPa | 130 MPa |
| Chemische Beständigkeit | 20,3 $\mu$g / cm$^2$ | 37 $\mu$g / cm$^2$ |

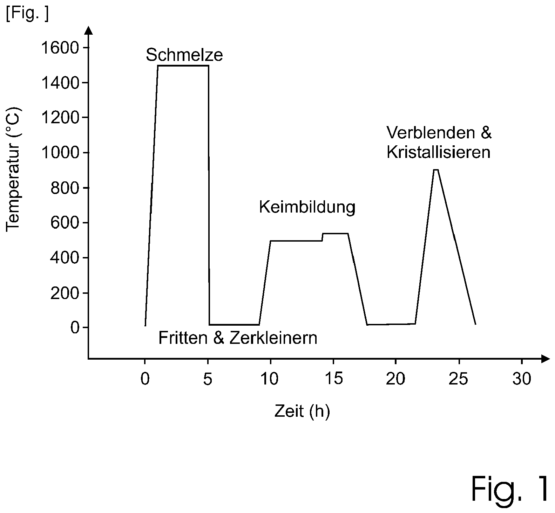

Für die Herstellung der Verblendkeramik wird in der Figur 1 ein technologischer Ablauf skizziert. Die Zusammensetzung der in der Tabelle 1 ausgewiesenen Ausgangsgläser wurde bei einer Temperatur von 1400°C in Platin - oder Keramiktiegeln erschmolzen und zur Herstellung einer Fritte in Wasser gegossen.

Zur Unterstützung der gesteuerten Kristallisation werden die gefritteten Ausgangsgläser ca. vier Stunden bei 540 ± 50°C getempert und nach dem Abkühlen pulverisiert. Die zum Einsatz gebrachte Korngröße liegt zwischen 0,3 $\mu$m und 30 $\mu$m.

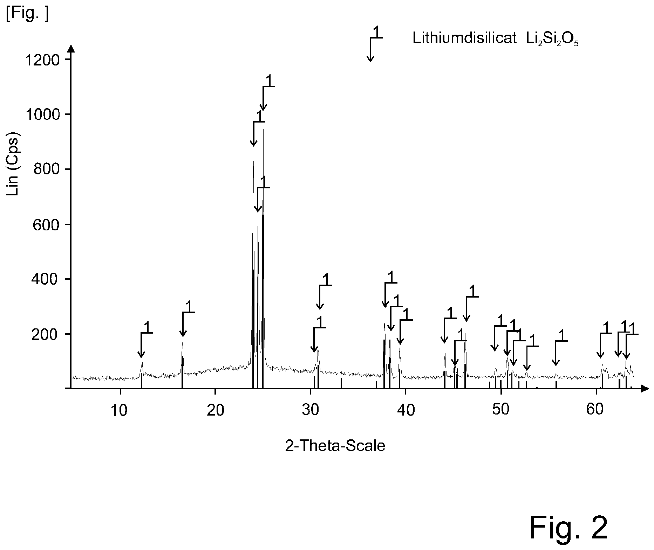

Die Kristallisation der Verblendkeramik beginnt bereits bei einer Temperatur von 760°C. Im Temperaturbereich von 760°C bis 840°C treten neben Lithiumdisilicat auch die Kristallphasen Lithiummetasilicat und Christobalit auf. Mit der Erhöhung der Temperatur lösen sich die Kristallphasen Lithiummetasilicat und Christobalit. Die gesteuerte Kristallisation erfolgt im Temperaturbereich von 850°C bis 940°C. Für die Kristallisationstemperatur von 900°C wird das Röntgendiffraktogramm (XRD) in der Figur 2 ausgewiesen.

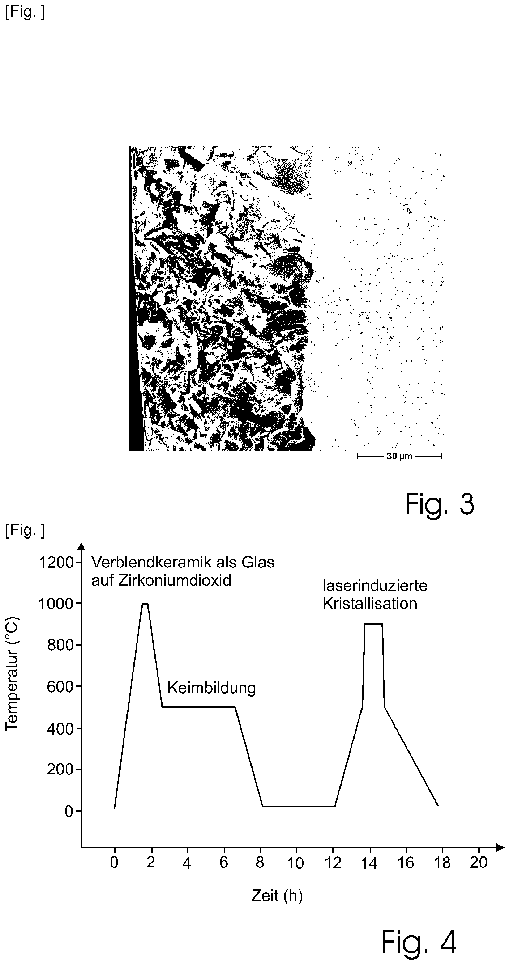

Während der Kristallisation bilden sich dünne Glasschichten sowohl an der Oberfläche als auch an der Grenzfläche zum yttriumstabilisiertem Zirkoniumdioxid. Die dünne Glasschicht an der Oberflächenschicht ist ein wichtiges Qualitätskriterium für die Verblendkeramik und ermöglicht rationellere Verarbeitungstechnologien. In der Figur 3 wird die Gefügestruktur der Verblendkeramik mittels Rasterelektronenmikroskopie abgebildet.

Das Auftragen der Verblendkeramik kann sowohl als Paste mittels Pinsel als auch über Sprühen erfolgen. Für den rationellen Auftrag dünner Schichten wird das Sprühen bevorzugt, wobei sowohl einschichtig als auch mehrschichtig gesprüht werden kann.

Ein anderer Verfahrensweg ist dadurch gegeben, dass die Verblendkeramik bei einer Temperatur von ca. 1000°C auf die Gerüstkeramik aus yttriumstabilisiertem Zirkoniumdioxid als transparentes Glas aufgeschmolzen wird. Dabei kann das Aufschmelzen des Glases sowohl über einen Keramikofen als auch durch den Einsatz eines Lasers erfolgen.

Die oberhalb der Liquidustemperatur auf das yttriumstabilisierte Zirkoniumdioxid aufgebrachte Verblendkeramik dient als Ausgangsmaterial für eine laserinduzierte Kristallisation in der Verblendkeramik. Durch den definierten Einsatz des Lasers ist es möglich, die aufgebrachten Schichten teilweise glasig zu gestalten oder in bestimmten Bereichen einer gezielten Kristallisation zu unterziehen. Ein möglicher Ablauf für die Kristallisation mittels Laser erfolgt gemäß Figur 4.

**Patentansprüche**

1. Verblendkeramik für dentale Restaurationen aus yttriumstabilisiertem Zirkoniumdioxid, **dadurch gekennzeichnet, dass** sie die folgenden Komponenten enthält:

   a) $SiO_2$ 55,0 - 72,5 Ma. %
   b) $Nb_2O_5$ 6,0 - 19,8 Ma. %
   c) $B_2O_3$ 1,0 - 9,0 Ma. %
   d) $Al_2O_3$ 1,2 - 6,0 Ma. %
   e) $Li_2O$ 5,0 - 16,5 Ma. %
   f) $Na_2O$ 1,4 - 11,0 Ma. %
   g) $ZrO_2$ 0,5 - 4,0 Ma. %.

2. Verblendkeramik für dentale Restaurationen aus yttriumstabilisiertem Zirkoniumdioxid, **dadurch gekennzeichnet, dass** sie die folgenden Komponenten enthält:

   a) $SiO_2$ 58,0 - 68,0 Ma. %
   b) $Nb_2O_5$ 10,0 - 19,8 Ma. %
   c) $Al_2O_3$ 1,2 - 6,0 Ma. %
   d) $Li_2O$ 8,0 - 16,5 Ma. %
   e) $Na_2O$ 1,4 - 8,0 Ma. %
   f) $ZrO_2$ 0,5 - 4,0 Ma. %.

3. Verblendkeramik nach Anspruch 1 oder 2, **dadurch gekennzeichnet, dass** mindestens eine der folgenden Komponenten $TiO_2$, $La_2O_3$, MgO, CaO, ZnO, BaO, $P_2O_5$ und/oder Fluorid zusätzlich enthalten ist, wobei die Gesamtkonzentration aller zugesetzten Komponenten maximal im Bereich bis zu 4,0 Ma. % liegt.

4. Verblendkeramik nach Anspruch 1 oder Anspruch 2, **dadurch gekennzeichnet, dass** als färbende oder fluoreszierende Zusätze Pigmente sowie die Oxide oder Fluoride der nachstehenden Elemente Fe, Sn, Ce, Mn, V, Cr, Pr, Tb, Nd, Sm, In, Eu, Dy einzeln oder in Kombination enthalten sind.

5. Verfahren zur Verblendung von dentalen Restaurationen aus yttriumstabilisiertem Zirkoniumdioxid mit einer Verblendkeramik nach einem der Ansprüche 1 bis 4 durch die folgenden Verfahrensschritte gekennzeichnet:

   a) ein bei 1400°C erschmolzenes Glas wird in Wasser gefrittet,
   b) das gefrittete Glas wird zur Keimbildung bei einer Temperatur von 490°C bis 590°C und einem Zeitraum von zwei bis sechs Stunden getempert,
   c) nach dem Abkühlen wird das getemperte Glas mechanisch bis auf eine Korngröße zwischen 0,3 $\mu$m und 30 $\mu$m pulverisiert,
   d) das vorbehandelte Pulver wird in eine Paste oder einen Schlicker überführt und auf die dentalen Restaurationen aufgetragen,
   e) eine Temperaturbehandlung im Bereich zwischen 850°C und 910°C wird durchgeführt, so dass in unmittelbarer Nähe zum auskristallisierten Lithiumdisilicat nioboxidreiche Phasen entstehen, wodurch an der Oberfläche die Bildung einer dünnen Glasschicht unterstützt wird, so dass kein gesonderter Glanzbrand erforderlich ist.

**6.** Verfahren nach Anspruch 5 **dadurch gekennzeichnet, dass** nach dem Verfahrensschritt e) mittels Laser eine lokale Verglasung der Verblendkeramik durchgeführt wird, um die Transluzenz bzw. Transparenz der Verblendkeramik zu erhöhen.

**7.** Verfahren nach Anspruch 5, **dadurch gekennzeichnet, dass** das vorbehandelte Pulver in eine Paste oder einen Schlicker durch Hinzugabe von Wasser und / oder organischen Hilfsmitteln überführt wird.

**8.** Verfahren nach Anspruch 5 bis 7, **dadurch gekennzeichnet, dass** das Auftragen des Schlickers durch Sprühen erfolgt.

**Claims**

**1.** Veneering ceramic for dental restorations comprising yttrium-stabilized zirconium dioxide, **characterized in that** it contains the following components:

a) $SiO_2$ 55.0 - 72.5 mass percent
b) $Nb_2O_5$ 6.0 - 19.8 mass percent
c) $B_2O_3$ 1.0 - 9.0 mass percent
d) $Al_2O_3$ 1.2 - 6.0 mass percent
e) $Li_2O$ 5.0 - 16.5 mass percent
f) $Na_2O$ 1.4 - 11.0 mass percent
g) $ZrO_2$ 0.5 - 4.0 mass percent.

**2.** Veneering ceramic for dental restorations comprising yttrium-stabilized zirconium dioxide, **characterized in that** it contains the following components:

a) $SiO_2$ 58.0 - 68.0 mass percent
b) $Nb_2O_5$ 10.0 - 19.8 mass percent
c) $Al_2O_3$ 1.2 - 6.0 mass percent
d) $Li_2O$ 8.0 - 16.5 mass percent
e) $Na_2O$ 1.4 - 8.0 mass percent
f) $ZrO_2$ 0.5 - 4.0 mass percent.

**3.** Veneering ceramic according to claim 1 or 2, **characterized in that** at least one of the following components is additionally contained: $TiO_2$, $La_2O_3$, MgO, CaO, ZnO, BaO, $P_2O_5$ and/or fluoride, wherein the total concentration of all of the added components is at most in the range of up to 4.0 mass percent.

**4.** Veneering ceramic according to claim 1 or claim 2, **characterized in that** pigments and the oxides or fluorides of the following elements are contained individually or in combination as colouring or fluorescing additions: Fe, Sn, Ce, Mn, V, Cr, Pr, Tb, Nd, Sm, In, Eu, Dy.

**5.** Method for veneering of dental restorations comprising yttrium-stabilized zirconium dioxide with a veneering ceramic according to one of claims 1 to 4, **characterized by** the following method steps:

a) a glass melted at 1400 ºC is fritted in water,
b) the fritted glass is tempered for nucleation at a temperature of 490 ºC to 590 ºC and for a duration of two to six hours,
c) after cooling, the tempered glass is mechanically pulverized to a grain size between 0.3 $\mu$m and 30 $\mu$m,
d) the pretreated powder is converted to a paste or a slip and applied to the dental restorations,
e) a heat treatment is carried out in the range between 850 ºC and 910 ºC so that niobium oxide-rich phases occur in close proximity to the crystallized lithium disilicate to promote formation of a thin glass layer on the surface so that no separate glaze firing is required.

**6.** Method according to claim 5, **characterized in that** the veneering ceramic is glazed locally by means of a laser after method step e) in order to increase the translucence or transparency of the veneering ceramic.

**7.** Method according to claim 5, **characterized in that** the pretreated powder is converted to a paste or a slip by the addition of water and/or organic agents.

8. Method according to claim 5 to 7, **characterized in that** the slip is applied by spraying.

### Revendications

1. Céramique de revêtement pour restaurations dentaires à base de dioxyde de zirconium stabilisé par l'yttrium, **caractérisée en ce qu'**elle contient les composants suivants:

   a) $SiO_2$ 55,0 - 72,5 % en masse
   b) $Nb_2O_5$ 6,0 - 19,8 % en masse
   c) $B_2O_3$ 1,0 - 9,0 % en masse
   d) $Al_2O_3$ 1.2 - 6,0 % en masse
   e) $Li_2O$ 5,0 - 16,5 % en masse
   f) $Na_2O$ 1,4 - 11,0 % en masse
   g) $ZrO_2$ 0,5 - 4,0 % en masse.

2. Céramique de revêtement pour restaurations dentaires à base de dioxyde de zirconium stabilisé par l'yttrium, **caractérisée en ce qu'**elle contient les composants suivants:

   a) $SiO_2$ 58,0 - 68,0 % en masse
   b) $Nb_2O_5$ 10,0 - 19,8 % en masse
   c) $Al_2O_3$ 1,2 - 6,0 % en masse
   d) $Li_2O$ 8,0 - 16,5 % en masse
   e) $Na_2O$ 1,4 - 8,0 % en masse
   f) $ZrO_2$ 0,5 - 4,0 % en masse.

3. Céramique de revêtement selon la revendication 1 ou 2, **caractérisée en ce qu'**au moins un des composants suivants est contenu en plus: $TiO_2$, $La_2O_3$, MgO, CaO, ZnO, BaO, $P_2O_5$ et/ou fluorure, la concentration totale de tous les composants ajoutés étant au maximum dans la plage allant jusqu'à 4,0 % en masse.

4. Céramique de revêtement selon la revendication 1 ou revendication 2, **caractérisée en ce que** des pigments et les oxydes ou fluorures des éléments suivants sont contenus individuellement ou en combinaison en tant qu'additifs colorants ou fluorescents: Fe, Sn, Ce, Mn, V, Cr, Pr, Tb, Nd, Sm, In, Eu, Dy.

5. Procédé de revêtement de restaurations dentaires à base de dioxyde de zirconium stabilisé par l'yttrium avec une céramique de revêtement selon une des revendications 1 à 4, **caractérisé par** les étapes du procédé suivantes:

   a) un verre fondu à 1400 $\underline{o}$C est fritté dans l'eau,
   b) le verre fritté est recuit pour la nucléation à une température de 490 $\underline{o}$C à 590 $\underline{o}$C et pour une durée de deux à six heures,
   c) après le refroidissement, le verre recuit est pulvérisé de manière mécanique jusqu'à une taille de grain entre 0,3 $\mu$m et 30 $\mu$m,
   d) le poudre prétraité est converti en une pâte ou une barbotine et appliqué sur les restaurations dentaires,
   e) un traitement thermique est effectué dans la plage entre 850 $\underline{o}$C et 910 $\underline{o}$C, de telle façon que des phases riches en oxyde de niobium sont formées à proximité immédiate du disilicate de lithium cristallisé pour soutenir la formation d'une mince couche de verre sur la surface de manière qu'une cuisson de glaçage séparée n'est pas nécessaire.

6. Procédé selon la revendication 5, **caractérisé en ce que** la céramique de revêtement est vitrée localement au moyen d'un laser après l'étape du procédé e) pour augmenter la translucidité ou transparence de la céramique de revêtement.

7. Procédé selon la revendication 5, **caractérisé en ce que** le poudre prétraité est converti en une pâte ou une barbotine en ajoutant de l'eau et/ou des auxiliaires organiques.

8. Procédé selon la revendication 5 à 7, **caractérisé en ce que** la barbotine est appliquée par pulvérisation.

Fig. 1

[Fig. ]

Fig. 2

[Fig. ]

30 µm

Fig. 3

[Fig. ]

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4798536 A
- US 4515634 A
- DE 19725552 A1
- DE 102004013455 B3
- DE 19647739 C2
- DE 19750794 A1
- US 20050127544 A1
- WO 2011076422 A1
- DE 10336913 A1
- WO 2008106958 A2
- EP 1235532 A1